# EUROPÄISCHE PATENTSCHRIFT

(11) **EP 0 380 681 B1**
(45) Veröffentlichungstag und Bekanntmachung des Hinweises auf die Patenterteilung: **28.08.1996**
(21) Anmeldenummer: 89905824.2
(22) Anmeldetag: 14.04.1989
(51) Int. Cl.: A61N 2/04

(54) **MAGNETOTHERAPIE-ANORDNUNG**
MAGNETOTHERAPY DEVICE
DISPOSITIF DE MAGNETOTHERAPIE

(30) Priorität: 25.04.1988 SU 4408261
(43) Veröffentlichungstag der Anmeldung: 08.08.1990
(73) Patentinhaber: 2 MOSKOVSKY GOSUDARSTVENNY MEDITSINSKY INSTITUT IMENI N.I.PIROGOVA, Moscow, 117869 (RU); NAUCHNO-ISSLEDOVATELSKY INSTITUT MEDITSINSKOI RADIOLOGII AKADEMII MEDITSINSKIKH NAUK SSSR, Obninsk, 249020 (RU); VSESOJUZNY NAUCHNO-ISSLEDOVATELSKY INSTITUT VETERINARNOI SANITARII, Moscow, 123022 (RU)
(72) Erfinder: TSYB, Anatoly Fedorovich, Obninsk, 249020 (SU); SERGEEV, Pavel Vasilievich, Moscow, 119121 (SU); SHARLAI, Vladimir Ivanovich, Obninsk, 249020 (SU); DIKUN, Vladimir Stepanovich, Minsk, 220077 (SU); SOBCHUK, Nikolai Sergeevich, Minsk, 220131 (SU); RAISKAYA, Tatyana Nikolaevna, Obninsk, 249020 (SU); KOTLYAR, Anatoly Dmitrievich, Minsk, 220061 (SU); BOLSHOV, Vladimir Mikhailovich, Moscow, 121359 (SU); TSARENKOV, Valery Minovich, Minsk, 220035 (SU); SULOEVA, Ljudmila Viktorovna, Minsk, 220073 (SU); BOGATOV, Oleg Gennadievich, Zheleznodorozhny, 143980 (SU)
(74) Vertreter: Lehn, Werner, Dipl.-Ing.
(86) Internationale Anmeldenummer: SU8900097
(87) Internationale Veröffentlichungsnummer: WO8910159

(56) Entgegenhaltungen:
- EP-A- 0 026 481
- EP-A- 0 048 451
- EP-A- 0 217 011
- EP-A- 0 244 046
- WO-A-85/02547
- CS-B- 0 252 197
- FR-A- 2 546 758
- SU-A- 0 697 131
- SU-A- 1 273 122
- US-A- 3 841 305
- US-A- 4 654 574
- SOVIET INVENTIONS ILLUSTRATED, Sektion EL,Derwntt publ.,London, GB; Woche 8750, Seite 8;& SU-1309274

## Beschreibung

### Gebiet der Technik

Die vorliegende Erfindung betrifft die Medizin und bezieht sich insbesondere auf eine Einrichtung zur Magnetotherapie.

### Zugrundeliegender Stand der Technik

Es ist eine Einrichtung zur Magnetotherapie bekannt, die -- in Reihe geschaltet -- einen Stromimpulsgenerator, einen Energiespeicher und einen Entladungskreis für den letzteren enthält, wobei der Entladungskreis ein steuerbares Schaltelement und einen Induktor aufweist, durch den ein heilendes Impulsmagnetfeld erzeugbar ist. Darüber hinaus enthält die Einrichtung eine Einheit zur Steuerung der Magnetfeldimpulsdauer des Induktors, deren Ausgang an den Steuereingang des Schaltelementes angeschlossen ist (SU, A, 1321426).

Die bekannte Einrichtung ist für die Betäubung bei Radikulitis, Plexitis und anderen Erkrankungen sowie für die Steuerung der Verschiebung von Röntgen-Kontrastmitteln im Organismus des Patienten bestimmt.Beim Einsatz der bekannten Einrichtung wählt der Arzt die für den Patienten geeignetste Amplitude und Dauer der Impulse eines heildenden Magnetfeldes des Induktors und eine Anzahl von Behandlungsschritten, während der auf den Kranken mit dem Magnetfeld eingewirkt wird. Dabei beträgt die für die Auswahl der optimalen Parameter magnetischer Impulse benötigte Zeit in der Regel mindestens 3 bis 7 Minuten. Während der Durchführung einer Behandlung der Magnetotherapie sind infolge Schwankungen der Charakteristiken der Baueinheiten der Einrichtung, hervorgerufen z. B. durch deren Erwärmung, Amplitudensprünge und Magnetfeldimpulse möglich, darunter auch auf Werte, die den zulässigen Wert übersteigen.

Bekannt ist ferner eine Einrichtung zur Magnetotherapie, die -- in Reihe geschaltet -- einen steuerbaren Stromimpulsgenerator, einen Energiespeicher und einen Entladungskreis für den letzteren enthält, welcher ein steuerbares Schaltelement und einen Induktor aufweist, durch den ein heilendes Impulsmagnetfeld erzeugbar ist, und die mit einer Einheit zur Steuerung der Magnetfeldimpulsdauer des Induktors, deren Ausgang an den Steuereingang des Schaltelementes angeschlossen ist, und einem Magnetfeldimpulsamplitudenregler versehen ist, dessen Ausgang an den Steuereingang des Impulsgenerators angeschlossen ist (EP, B, 0048451). Der Amplitudenregler dieser Einrichtung gewährleistet eine Begrenzung der Spitzenwerte der Amplitude der Magnetfeldimpulse. Die Auswahl der für den Patienten optimalen Parameter der Impulse eines heilenden Magnetfeldes wird jedoch bei Verwendung der letztgenannten Einrichtung durch längeres Variieren der Werte der Impulsamplitude und -dauer durchgeführt, was eine beträchtliche Zeit in Anspruch nimmt und sich nicht in allen Fällen als effektiv erweist. Insbesondere erweist sich als kompliziert die Auswahl der optimalen Parameter mit Hilfe der bekannten Einrichtung beim Kupieren eines akuten schmerzhaften Syndroms, von Phantomschmerzen, Bronchialasthmaanfällen.

### Offenbarung der Erfindung

Der Erfindung liegt die Aufgabe zugrunde, die Einrichtung zur Magnetotherapie so zu vervollkommnen, daß sie über Mittel zur Auswahl der Parameter und Form der Impulse eines heilenden Magnetfeldes verfügt, welche das Kupieren eines akuten schmerzhaften Syndroms, von Phantomschmerzen, Bronchialasthmaanfällen für einen konkreten Kranken in möglichst kurzen Zeiträumen sichern.

Des Wesen der Erfindung besteht darin, daß bei der Einrichtung zur Magnetotherapie, die -- in Reihe geschaltet -- einen steuerbaren Stromimpulsgenerator, einen Energiespeicher und einen Entladungskreis für denselben enthält, welcher ein steuerbares Schaltelement und einen Induktor, durch den ein heilendes Impulsmagnetfeld erzeugbar ist, aufweist, und die mit einer Einheit zur Steuerung der Magnetfeldimpulsdauer des Induktors, deren Ausgang an den Steuereingang des steuerbaren Schaltelementes angeschlossen ist, und einem Magnetfeldimpulsamplitudenregler versehen ist, dessen Ausgang an den Steuereingang des steuerbaren Stromimpulsgenerators angeschlossen ist, gemäß der Erfindung in den Entladungskreis des Energiespeichers ein Regelteil für die Vorderflankensteilheit der Magnetfeldimpulse des Induktors geschaltet ist.

Der Regelteil für die Vorderflankensteilheit der Magnetfeldimpulse des Induktors kann zwischen dem Ausgang des Energiespeichers und dem Eingang des Schaltelementes geschaltet sein und mindestens zwei LC-Ketten enthalten, deren eine Anschlußenden zusammengeschaltet sind, und einen Wahlschalter aufweisen, dessen Ruhekontakte mit den anderen Anschlußenden der entsprechenden LC-Ketten verbunden sind.

Man kann, wenn der Induktor eine mehrwindige Induktivitätsspule darstellt, mit dieser den Regelteil für die Vorderflankensteilheit der Magnetfeldimpulse konstruktiv vereinigen, wozu die Wicklung der Induktivitätsspule mit einer Vielzahl von Abzweigen über die Länge des Wicklungsleiters zu versehen und die Spule an den Ausgang des Schaltelementes über einen Wahlschalter anzuschließen ist, dessen jeder Ruhekontakt mit dem zugehörigen Abzweig verbunden ist.

Um die Konstanz der Impulsamplitude bei Änderung der Impulsvorderflanke zu sichern, erweist es sich als zweckmäßig bei der Einrichtung zur Magnetotherapie, in der der Magnetfeldimpulsamplitudenregler eine Vergleichsschaltung enthält, deren erster Eingang an den Ausgang eines Magnetfeldimpulsamplitudengebers, zweiter Eingang an den Ausgang eines Magnetfeldimpulsamplitudeneinstellers und deren Ausgang an den Ausgang des Magnetfeldimpulsamplitudenreglers angeschlossen sind, in den Amplitudenregler eine zwischen dem Ausgang des Magnetfeldimpulsamplitudengebers und dem ersten Eingang der Vergleichsschaltung geschaltete Speichereinheit und eine zweite Vergleichsschaltung zusätzlich einzuführen, deren erster Eingang mit dem Ausgang der ersten Vergleichsschaltung, zweiter Eingang über einen Spannungsteiler mit dem Ausgang des Energiespeichers und deren Ausgang mit dem Ausgang des Amplitudenreglers verbunden sind.

Die erfindungsgemäß ausgeführte Einrichtung zur Magnetotherapie bietet durch Auswahl eines optimalen Wertes der Vorderflankenanstiegszeit dem Impulses eines heilenden Magnetfeldes die Möglichkeit, einen akuten schmerzhaften Syndrom, Phantomschmerzen, einen Bronchialasthmaanfall während erster drei Minuten vom Anfang der Einwirkung an zu kupieren. Die Verwendung der erfindungsgemäßen Einrichtung garantiert die Beibehaltung des Schmerzbetäubungseffektes im Laufe von 6 bis 12 Stunden nach dem Aufhören der magnetotherapeutischen Heilung.

### Kurzbeschreibung der Zeichnungen

Nachstehend wird die Erfindung anhand von konkreten Ausführungsformen unter Bezugnahme auf die beigelegten Zeichnungen näher erläutert. Es zeigt:
Fig. 1 ein Strukturschaltbild einer erfindungsgemäßen Einrichtung zur Magnetotherapie;
Fig. 2 ein Strukturschaltbild einer Ausführungsform der Einrichtung zur Magnetotherapie, bei der der Induktor und der Regelteil für die Vorderflankensteilheit der Magnetfeldimpulse konstruktiv miteinander vereinigt sind;
Fig. 3 eine elektrische Prinzipschaltung des Regelteils für die Vorderflankensteilheit der Magnetfeldimpulse;
Fig. 4 die Gesamtansicht des Induktors (im Längsschnitt), der mit dem Regelteil für die Vorderflankensteilheit der Magnetfeldimpulse vereinigt ist;
Fig. 5 einen Schnitt entlang der Linie V-V in Fig. 4;
Fig. 6 eine elektrische Prinzipschaltung für den Stromimpulsgenerator und den Energiespeicher;
Fig.7 ein Strukturschaltbild eines Amplitudenreglers;
Fig. 8 eine Abhängigkeit der magnetischen Feldstärke H des Induktors von der Zeit (t), wobei auf der Ordinatenachse magnetische Feldstärken H in [kA/m] und auf der Abszissenachse Werte der Zeit t in [s] aufgetragen sind.

### Bevorzugte Ausführungsform der Erfindung

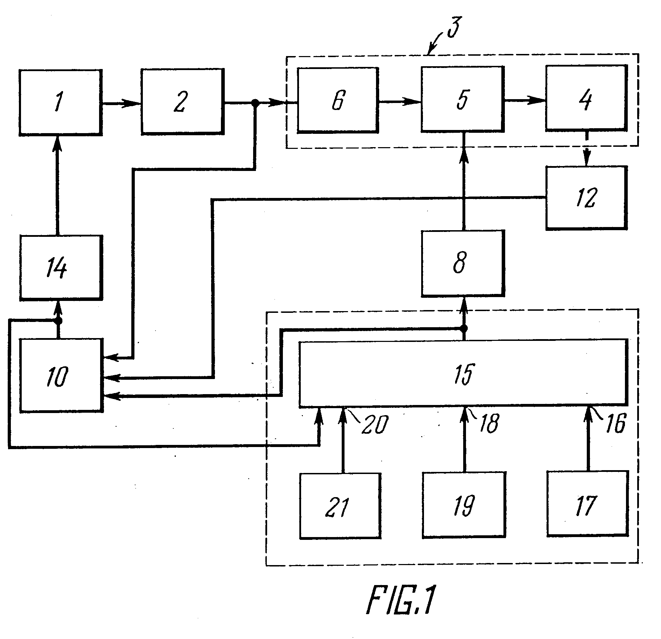

Die in Fig. 1 gezeigte Einrichtung zur Magnetotherapie enthält -- in Reihe geschaltet -- einen steuerbaren Stromimpulsgenerator 1, einen Energiespeicher 2 und einen Entladungskreis 3 für den Speicher 2. Der Entladungskreis 3 weist einen Induktor 4 und einen an den Eingang des letzteren über ein steuerbares Schaltelement 5 angeschlossenen Regelteil 6 für die Vorderflankensteilheit der Magnetfeldimpulse des Induktors auf, dessen Eingang seinerseits am Ausgang des Energiespeichers 2 liegt. Die Einrichtung zur Magnetotherapie ist auch mit einer Einheit 7 zur Steuerung der Magnetfeldimpulsdauer versehen, deren Ausgang über einen Leistungsverstärker 8 an den Steuereingang des Schaltelementes 5 und an den Eingang 9 eines Magnetfeldimpulsamplitudenreglers 10 gelegt ist, dessen Eingang 11 am Ausgang eines Magnetfeldimpulsamplitudengebers 12 liegt und dessen Eingang 13 mit dem Ausgang des Energiespeichers 2 verbunden ist, wobei der Ausgang des Reglers 10 über einen Leistungsverstärker 14 an den Steuereingang des Stromimpulsgenerators 1 angeschlossen ist. Die Einheit 7 zur Steuerung der Magnetfeldimpulsdauer enthält einen Impulsgenerator 15 mit umstimmbarer Impulsfrequenz und -dauer, bei dem an den Eingang 16 ein Impulsfrequenzeinsteller 17, an den Eingang 18 ein Impulsdauereinsteller 19, an den Eingang 20 ein Impulszahleinsteller 21 und an den Steuereingang der Ausgang des Magnetfeldimpulsamplitudenreglers 10 angeschlossen sind.

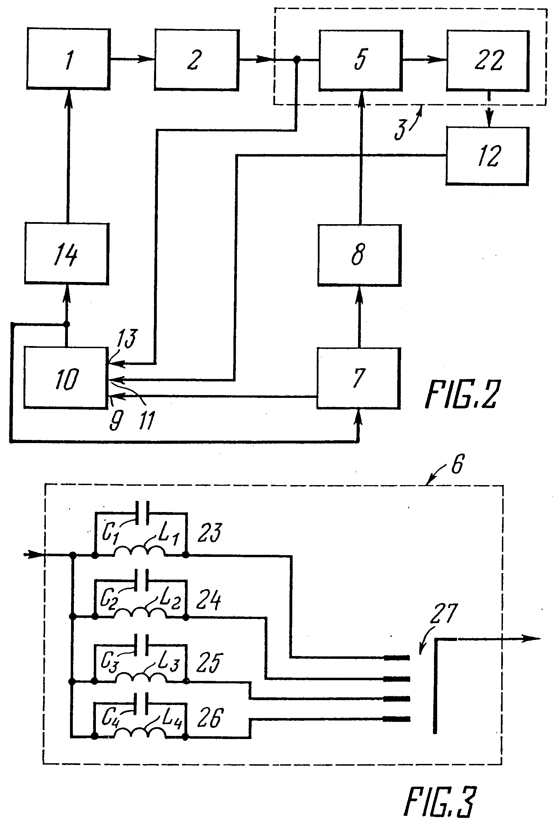

In Fig. 2 ist ein Strukturschaltbild einer Ausführungsvariante der Einrichtung zur Magnetotherapie gezeigt, welches sich von dem in Fig. 1 dargestellten dadurch unterscheidet, daß deren Induktor 22 mit dem Regelteil für die Vorderflankensteilheit der Magnetfeldimpulse vereinigt ist. Hierbei entfällt im Entladungskreis 3 ein separater Regelteil für die Vorderflankensteilheit, und der Entladungskreis 3 enthält einen Induktor 22, dessen Eingang über das steuerbare Schaltelement 5 unmittelbar an den Ausgang des Energiespeichers 2 geschaltet ist.

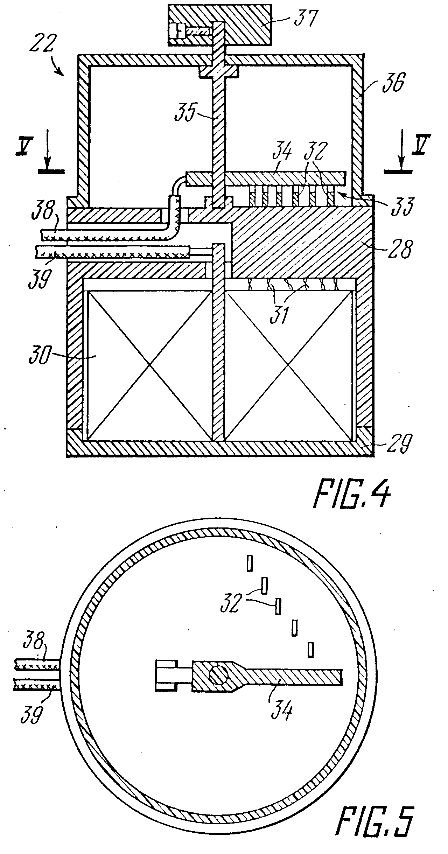

Ist die Einrichtung zur Magnetotherapie nach dem in Fig. 1 gezeigten Schaltbild ausgeführt, so kann der Regelteil 6 für die Vorderflankensteilheit der Magnetfeldimpulse eine elektrische Schaltung haben, die in Fig. 3 dargestellt ist. Bei der vorliegenden Ausführungsvariante besteht er aus vier LC-Parallelkreise 23, 24, 25, 26, die im allgemeinen Fall verschiedene L- bzw. C-Werte haben, wobei deren Eingänge zusammengeschaltet und an den Ausgang des Energiespeichers 2 angeschlossen sind und deren Ausgänge mit den Ruhekontakten eines Wahlschalters 27 in Verbindung stehen, dessen gemeinsamer beweglicher Kontakt mit dem Eingang des steuerbaren Schaltelementes 5 verbunden ist.

In Fig. 3 ist eine Variante des Regelteils 6 für die Vorderflankensteilheit der Magnetfeldimpulse gezeigt, bei der LC-Parallelkreise verwendet werden. Mit demselben Erfolg kann jedoch der Regelteil 6 unter Verwendung von LC-Reihenketten oder unter gleichzeitiger Verwendung von LC-Reihen- und LC-Parallelketten ausgeführt werden, wobei er nur die Möglichkeit geben müßte, die Ersatzinduktivität L und die Ersatzkapazität C des Regelteils 6 in den geforderten Grenzen zu verändern.

Als Induktor 4 kann eine mehrwindige Induktivitätsspule benutzt sein, die imstande ist, auf der Arbeitsfläche des Induktors ein zureichend homogenes Impulsmagnetfeld mit einer Feldstärke von 2000 kA/m zu erzeugen.

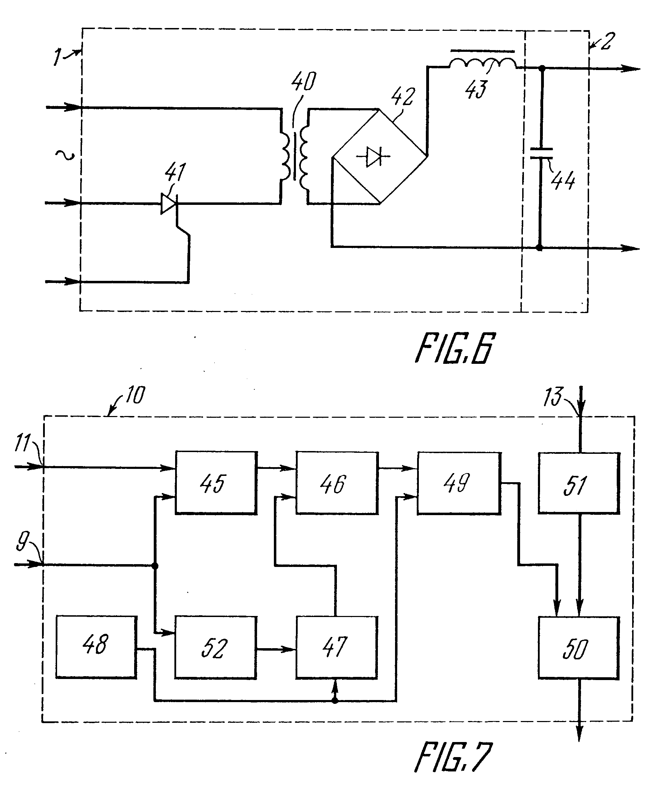

In Fig. 4, 5 ist eine Ausführungsvariante des Induktors 22 wiedergegeben, bei der der eigentliche Induktor 22 und der Regelteil für die Vorderflankensteilheit der Magnetfeldimpulse konstruktiv vereinigt sind.

Der Induktor 22 enthält ein zylindrisches Hohlgehäuse 28 aus einem dielektrischen Material,welches mit einem abnehmbaren Deckel 29 versehen ist. Im Hohlraum des Gehäuses 28 ist gleichachsig mit diesem eine mehrwindige Induktivitätsbandspule 30 mit einer spiralen Wicklung untergebracht, die mit einer Vielzahl von Abzweigen 31 über die Länge des Wicklungsleiters - im hier zu behandelnden Ausführungsform mit sechs Abzweigen - versehen ist. Jeder Abzweig 31 ist mit einem zugehörigen Ruhekontakt 32 eines Lamellen-Wahlschalters 33 verbunden, dessen gemeinsamer beweglicher Kontakt 34 an einer Zentralachse 35 befestigt ist, die im Gehäuse 28 drehbar gelagert ist. Der Wahlschalter 33 ist durch einen Deckel 36 geschützt, an dessen Außenfläche ein Handgriff 37 angebracht ist, der mit dem über den Deckel 36 hinausragenden Ende der Achse 35 fest verbunden ist und zur Drehung der Achse 35 zusammen mit dem gemeinsamen Kontakt 34 um einen vorgegebenen Winkel dient. Der gemeinsame Kontakt 34 ist mit einer Stromzuleitung 38 verbunden, während eines der Enden der Wicklung der Spule 30, im vorliegenden Fall das innere Ende, mit einer Stromzuleitung 39 verbunden sind. Die Stromzuleitungen 38 und 39 sind aus dem Gehäuse 28 herausgeführt.

Fig. 6 zeigt eine elektrische Schaltung für den Stromimpulsgenerator 1 mit dem Energiespeicher 2. Der Stromimpulsgenerator 1 enthält einen Transformator 40, einen Symistor 41, der in den Primärwicklungskreis des Transformators 40 in Reihe geschaltet und über den als Steuereingang des Impulsgenerators 1 dienenden Steuereingang mit dem Leistungsverstärker 14 verbunden ist, eine Diodengleichrichterbrücke 42, deren Eingänge an die Sekundärwicklung des Transformators 40 angeschlossen sind. An den Ausgang der Diodengleichrichterbrücke 42 ist eine Strombegrenzungsdrossel 43 und ein Energiespeicher 2 hintereinandergeschaltet, der in Form einer Batterie von Kondensatoren ausgeführt ist, die einen Entladungskreis bilden, durch den beim Betrieb der Einrichtung ein Ladestrom periodisch fließt. Die Kondensatorbatterie 44 kann auf Grundlage von Impulskondensatoren mit geringer Eigeninduktivität ausgeführt sein. Das Schaltelement 5 stellt in der gegebenen Ausführungsvariante einen Symistor dar.

Als Magnetfeldimpulsgeber 12 dient ein Hall-Kristallwandler.

Als Leistungsverstärker 8 und 14 können Emitterfolger benutzt werden, die jeweils auf einem zusammengesetzten Transistor aufgebaut sind.

Als Impulsgenerator 15 kann z. B. ein weit bekannter NF-Impulsgenerator benutzt sein, der auf Mikroschaltkreisen aufgebaut ist und bei dem die Impulsfrequenz und -dauer durch Änderung der Parameter der frequenzbestimmenden LC-Kette auf die Signale des Impulsfrequenz- und des Impulsdauereinstellers 17 bzw. 19 geändert werden, als welche Wahlschalter verwendet werden.

Der Impulszahleinsteller 21 stellt einen Dekaden-Wahlschalter dar, während in die Schaltung des Impulsgenerators 15 außerdem ein Impulszähler und ein Digitalkomparator geschaltet sind. Dabei sind die Ausgänge des Dekaden-Wahlschalters mit einer Gruppe von Eingängen des Digitalkomparators verbunden, während die Ausgänge des Impulszählers mit der anderen Gruppe von Eingängen des gleichen Digitalkomparators (nicht gezeigt) in Verbindung stehen.

Der Magnetfeldimpulsamplitudenregler 10, dessen Strukturschaltbild in Fig. 7 dargestellt ist, enthält eine Speichereinheit 45, deren einer Eingang an den Eingang 11 des Reglers 10 und deren anderer Eingang an den Eingang 9 des Reglers 10 angeschlossen ist, eine Vergleichsschaltung 46, deren erster Eingang mit dem Ausgang der Speichereinheit 45 und deren zweiter Eingang über einen steuerbaren Schalter 47 mit dem Ausgang eines Magnetfeldimpulsamplitudeneinstellers 48 in Verbindung steht, einen Summator 49, dessen erster Eingang an den Ausgang der Vergleichsschaltung 46 gelegt ist und dessen zweiter Eingang am Ausgang des Magnetfeldimpulsamplitudeneinstellers 48 unmittelbar liegt, eine Vergleichsschaltung 50, deren erster Eingang an den Ausgang des Summators 49 und deren zweiter Eingang über einen Spannungsteiler an den Eingang 13 des Reglers 10 angeschlossen ist und deren Ausgang den Ausgang des Magnetfeldimpulsaplitudenreglers 10 bildet, sowie eine Steuerachaltung 52 für den Schalter 47, deren Eingang an den Eingang 9 des Reglers 10 und deren Ausgang an den Steuereingang des Schalters 47 gelegt ist. Die Bauteile 45, 46, 49, 50 sind auf der Basis von Operationsverstärkern aufgebaut. Der Amplitudeneinsteller 48 stellt einen Spannungsteiler dar, der unter Verwendung eines Stellwiderstandes ausgeführt ist.

Die Steuerschaltung 52 des Schalters 47 kann auf der Basis eines RS-Flipflops aufgebaut werden.

Die erfindungsgemäße Einrichtung zur Magnetotherapie arbeitet wie folgt.

Bevor man die Einrichtung ans Versorgungsnetz anschließt, stellt man - beruhend auf ärztlicher Erfahrung - mit Hilfe des Impulsfrequenz-, Impulsdauer- und Impulszahleinstellers 17 (Fig. 1), 19 bzw. 31 sowie des Magnetfeldimpulsamplitudeneinstellers 43 (Fig. 7) des Amplitudenreglers 10 und des Wahlschalters 27 (Fig 3) die für die meisten Kranken mit irgendeiner Pathologie effektivste Betriebsart der Einrichtung zur Magnetotherapie ein, d. h. es werden Frequenz, Dauer, Amplitude, Vorderflankensteilheit der Impulse des Magnetfeldes und deren Zahl für eine Heilseance vorgegeben. Der Induktor 4 (Fig. 1) wird zusammen mit dem Magnetfeldimpulsamplitudengeber 12, der in die dem zu beeinflussenden Objekt zugewandte untere Grundfläche des zylindrischen Induktors eingebaut ist, auf ein Gebiet vermutlicher therapeutischer Einwirkung so gestellt, daß die Arbeitsfläche des Induktors 4 (Stirnseite der Inuktivitätsspule) mit dem Körper des Patienten in Berührung kommt oder sich in unmittelbarer Nähe desselben befindet. Bei Zuführung der Spannung des Versorgungsnetzes an den Eingang der Einrichtung zur Magnetotherapie beginnt die Kondensatorenbatterie 44 (Fig.6) des Energiespeichers 2 sich aufzuladen, weil der Symistor 41 im Ausgangszustand leitend ist, so daß der Ladestrom die Primärwicklung des Transformators 40 durchfließt. Dabei tritt an der Sekundärwicklung des Transformators 40 eine Wechselspannung auf, die in der Diodengleichrichter 42 in eine Gleichspannung umgewandelt wird. Die Drossel 43 dient der Begrenzung des Ladestroms. Eine Information, die dem am Energiespeicher 2 liegenden Spannungswert zugeordnet ist, gelangt von dessen Ausgang zum Eingang 13 (Fig. 1) des Magnetfeldimpulsamplitudenreglers 10. Sobald die Spannung am Energiespeicher 2 einen Wert erreicht hat, der für die Gewinnung eines Magnetfeldimpulses mit der Amplitude ausreichend ist, die durch den Amplitudeneinsteller 48 (Fig. 7) vorgegeben ist, liefert der Regler 10 ein Signal, durch das der Symistor 41 (Fig. 6) gesperrt wird, wodurch die Aufladung des Energiespeichers 2 beendet ist. Der Energiespeicher 2 ist zur Entladung bereit. Gleichzeitig gelangt vom Ausgang des Reglers 10 (Fig. 1) zum Eingang des Impulsgenerators 15 ein Signal, durch das die Abgabe eines Impulses von der Einheit 7 zur Steuerung der Magnetfeldimpulsdauer an den Steuereingang des Schaltelementes 5 freigegeben wird. Das Schaltelement 5 schaltet den Entladungskreis 3 an den Ausgang des Energiespeichers 2, so daß die Entladung des Speichers 2 über den Entladungskreis 3 erfolgt, der mit dem Regelteil 6 für die Vorderflankensteilheit der Magnetfeldimpulse versehen ist, durch den in den Entladungskreis 3 mit Hilfe des Wahlschalters 27 (Fig. 3) eine der LC-Ketten 23 bis 26, z. B. die LC-Kette 23 eingeschaltet ist. Das Umschalten der LC-Ketten 23 bis 26 im Regelteil 6 führt zu einer Änderung der Ersatzgrößen des Widerstandes Rₒ, der Induktivität Lₒ, der Kapazität Cₒ des Entladungskreises 3 (Fig. 1), durch welche die Parameter der Magnetfeldimpulse des Induktors 4, insbesondere die Vorderflankenanstiegszeit τ_{f} des Magnetfeldimpulses festgelegt werden, die die Vorderflankensteilheit des Impulses bestimmt.

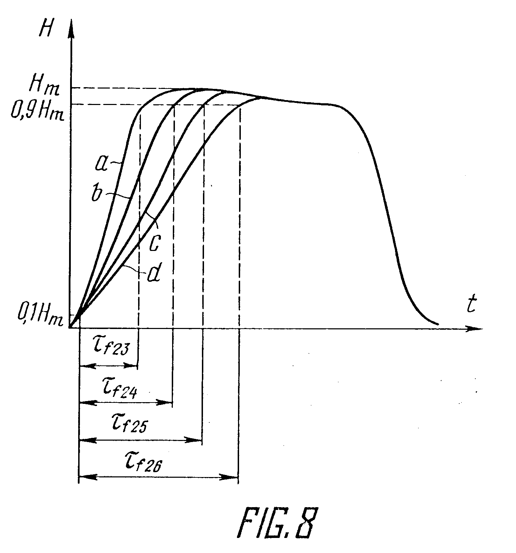

In Fig. 8 sind Verläufe für einen Magnetfeldimpulses des Induktors 4 (Fig. 1) bei verschiedenen Schaltungsarten der LC-Ketten 23 bis 26 (Fig. 3) im Regelteil 6 für die Vorderflankensteilheit dargestellt.

Die Kurve "a" stellt einen Impulsverlauf beim Anschluß der LC-Kette 23 an das Schaltelement 5 (Fig. 1) über den Wahlschalter 27, die Kurve "b" beim Anschluß der LC-Kette 24, die Kurve "c" beim Anschluß der LC-Kette 25 und die Kurve "d" beim Anschluß der LC-Kette 26 dar.

Da die Steilheit der Impulsvorderflanke durch das Verhältnis der Magnetfeldimpulsamplitude zur Vorderflankenanstiegszeit τ_{f} bestimmt wird, führt eine Änderung der Zeit τ_{f} zu einer Änderung der Vorderflankensteilheit der Magnetfeldimpulse, und auf diese Weise wird die Regelung der Vorderflankensteilheit der Magnetfeldimpulse verwirklicht.

Der Magnetfeldimpulsamplitudenregler 10 (Fig. 10) funktioniert folgenderweise.

Nach Einschalten der Einrichtung zur Magnetotherapie wird, bevor der erste Entladungsimpuls des Energiespeichers geliefert und in die Speichereinheit 45 ein Signal eingeschrieben werden soll, das der tatsächlichen Amplitude des Magnetfeldimpulses zugeordnet ist und vom Magnetfeldimpulsamplitudengebern 12 (Fig. 1) eintrifft, an den ersten Eingang der Vergleichsschaltung 46 (Fig. 7) ein Nullsignal angelegt. Am zweiten Eingang der Schaltung 46 liegt ebenfalls das Nullsignal, weil kein Signal vom Magnetfeldimpulsamplitudeneinsteller 48 den steuerbaren Schalter 47 durchläuft, der mit einem von der Steuerschaltung 52 eintreffenden Signal gesperrt ist. Dadurch liefert die Vergleichsschaltung 46 ein Nullsignal, das zu einem Eingang des Summators 49 gelangt. Am zweiten Eingang des Summators 49 trifft ein Signal vom Magnetfeldimpulsamplitudeneinsteller 48 ein. Vom Ausgang des Summators 49 wird das dem Signal vom Amplitudeneinsteller 48 gleiche Signal der Vergleichsschaltung 50 zugeführt, in der es mit einem Signal verglichen wird, welches durch den Spannungsteiler 51 über den Eingang 13 vom Energiespeicher 2 (Fig. 1) gelangt und eine Information über den Wert der an diesem Speicher liegenden Spannung repräsentiert.

Sind die Werte der Signale an den Eingängen der Schaltung 50 (Fig. 7) gleich, so wird an deren Ausgang ein Signal eingestellt, mit dem über den Leistungsverstärker 14 (Fig. 1) der Symistor 41 (Fig. 6) gesperrt und das Aufhören der Aufladung der Kondensatorenbatterie 44 des Energiespeichers 2 bewirkt wird.

Zu demselben Augenblick kommt vom Ausgang der Einheit 7 (Fig. 1) am Eingang des Amplitudenreglers 10 ein Signal an, das an den Eingang der Speichereinheit 45 (Fig. 7) weitergegeben wird, die letztere in einen Zustand bringt, bei dem sie bereit ist, Impulse vom Impulsamplitudengeber 12 (Fig. 1) aufzunehmen, sowie der Steuerschaltung 52 (Fig. 7) zugeführt wird, an deren Ausgang ein Signal gebildet wird, das den Schalter 47 über den Steuereingang entsperrt. Dabei befindet sich der Schalter 47 im leitenden Zustand vom Zeitpunkt der Ankunft des ersten Impulses bis zu einem Zeitpunkt, in dem die Baugruppen der Einrichtung nach der Erzeugung der Anzahl der Impulse in den Ausgangszustand gebracht werden, die am Impulszahleinsteller 21 (Fig. 1) der Einheit 7 voreingestellt ist. Während der Entladung des Energiespeichers 2 (Impulszeit) wird in die Speichereinheit 45 (Fig. 7) eine Information vom Amplitudengeber 12 (Fig. 1) eingeschrieben, welche Daten über den Istwert der Amplitude des Magnetfeldimpulses des Induktors 4 enthält.

Somit werden den Eingängen der Vergleichsschaltung 46 (Fig. 7) ein dem Istwerk der Amplitude des Magnetfeldimpulses entsprechendes Signal und ein dem Sollwert der Amplitude zugeordnetes Signal vom Magnetfeldimpulsamplitudeneinsteller 48 über den leitenden Schalter 47 zugeführt. In der Vergleichsschaltung 46 werden die Soll- und die Istamplitude des Magnetfeldimpulses verglichen und ein Abweichungssignal geliefert. Dieses gelangt zum ersten Eingang des Summators 49, wo es je nach Vorzeichen den Signalwert vom Amplitudeneinsteller 48 (vergrößert oder verkleinert) ändert, welcher dem zweiten Eingang des Summators 49 zugeführt wird, von dessen Ausgang das Signal an den ersten Eingang der Vergleichsschaltung 50 angelegt wird. Dieses Signal ist im wesentlichen dasjenige Signal, das die Amplitude des Magnetfeldimpulses unter Berücksichtigung der Korrektur von Störgrößen vorgibt, die in den Entladungskreis 3 (Fig. 1), insbesondere bei der Umschaltung der LC-Ketten 23 bis 26 (Fig. 3) im Regelteil 6, hineingebracht werden. Dadurch wird die Amplitude der Magnetfeldimpulse des Induktors 4 konstant gehalten. Ist beispielsweise die tatsächliche Amplitude des Magnetfeldimpulses höher als die Sollamplitude, dann liegt am Eingang der Vergleichsschaltung 50 ein Signalpegel an, der geringer als der vom Impulsamplitudeneinsteller 48 ist. Daher wird das am zweiten Eingang der Vergleichsschaltung 50 eintreffende Signal vom Spannungsteiler 51 gleich dem am ersten Eingang liegenden Signal bei einer geringeren Spannung am Energiespeicher 2 (Fig. 1). Da die Spannung am Energiespeicher 2 geringer wurde, wird die Amplitude des Magnetfeldimpulses bei der Entladung des Speichers 2 über den Entladungskreis 3 vermindert, d. h. sie wird gleich dem Sollwert.

Im weiteren wird das Arbeitsspiel der Einrichtung wiederholt. Nachdem die Anzahl der von der Einrichtung zur Magnetotherapie erzeugten Magnetfeldimpulse den vom Einsteller 21 vorgegebenen Sollwert erreicht hat, wird die Funktion der Einrichtung unterbrochen und diese in den Ausgangszustand gebracht. Die Parameter des Entladungskreises 3 des Energiespeichers 2 sind so bemessen, daß bei der Arbeit auf der höchsten Frequenz der Energiespeicher 3 zum Aufladen auf den Höchstwert Zeit hat.

Ahnlich arbeitet auch die Einrichtung zur Magnetotherapie, deren Schaltbild in Fig. 2 gezeigt ist. Der Unterschied besteht darin, daß die Flankensteilheit der Magnetfeldimpulse durch Änderung der Induktivität des Induktors 22 selbst geregelt wird. Durch Änderungen der Parameter des Induktors 22 und durch Umschaltungen im Regelteil 6 (Fig. 1) ergibt sich eine Änderung der Parameter des Entladungskreises 3, welche auch die Amplitude der Magnetfeldimpulse beeinflussen. Jedoch wird diese Änderung vom Magnetfeldimpulsamplitudengeber 12 erfaßt, und der Regler 10 kompensiert diese Änderung, so daß die Amplitude der Magnetfeldimpulse gleich dem Sollwert Hₘ aufrechterhalten wird.

Die erfindungsgemäße Einrichtung zur Magnetotherapie sorgt für die Erzeugung magnetischer Impulse an der Arbeitsfläche des Induktors, wobei sie eine Dauer von 10⁻² bis 10⁻⁶ s, eine Amplitude von 500 bis 2000 kA/m und eine Folgefrequenz von 0,25 bis 2 Hz aufweisen.

Der Vorteil der erfindungsgemäßen Einrichtung besteht in der Möglichkeit einer dynamischen Steuerung des therapeutischen Behandlungsvorganges. Durch Regelung der Vorderflankensteilheit magnetischer Impulse während der Einwirkung auf den Kranken kann man einen Wert des angegebenen Parameters entsprechend der individuellen Empfindlichkeit des Organismus auswählen und einen Schmerzbetäubungseffekt in einer optimal kurzen Zeitspanne erreichen.

Die erfindungsgemäße Einrichtung ermöglicht eine Änderung der Vorderflankensteilheit der Impulse in Grenzen von 5.10⁵ bis 2.10⁸ kA/m.s. Durch Erhöhung der Vorderflankensteilheit der Impulse und durch die Regelbarkeit dieser Steilheit wird der therapeutische Behandlungseffekt verbessert, und zwar kann man bei der Behandlung der lumbosakralen Radikulitis bei einmaliger Einwirkung die Schmerzbetäubungszeit durchschnittlich um ein 2,5faches verkürzen und gleichzeitig damit die Beibehaltungszeit des Schmerzbetäubungseffektes im Mittel um ein 4,5faches verlängern.

Durch die automatische Stabilisierung der Magnetfeldamplitude wird manuelle Überwachung und Konstanthaltung der Magnetfeldamplitude vermieden, was den Behandlungsvorgang vereinfacht und den Zeitraum für die Durchführung eines magnetotherapeutischen Behandlungsschrittes zu verkürzen.

Es wurde ein Versuchsmuster der erfindungsgemäßen Einrichtung zur Magnetotherapie unter klinischen Verhältnissen hergestellt und geprüft.

Nachstehend werden Untersuchungsergebnisse aufgeführt.

Man benutzte die erfindungsgemäße Einrichtung zur Magnetotherapie zum Kupieren von akuten posttraumatischen Schmerzen in Abschnitten, wo eine Nekrose bei einem Kranken mit eitrigen und nekrotischen Unterschenkelwunden ausgeschnitten worden ist. Man stellte eine Amplitude der Impulse des heilenden Magnetfeldes von 1300 kA/m, eine Impulsdauer von 3.10⁻⁴ s, eine Anstiegsgeschwindigkeit der Impulsvorderflanke von 2,6.10⁷kA/ms ein. Nach der Einwirkung mit 50 Impulsen (∼ 2,5 min) trat beim Kranken ein Zustand subjektiver Betäubungsempfindung ein. Nach der Durchführung eines magnetotherapeutischen Behandlungsschrittes mit 100 Impulsen wurde der Schmerzbetäubungseffekt 6 Stunden lang beibehalten. Nach fünf Behandlungsschritten mit je 100 Impulsen heilten die Wunden ab und die Schmerzen sind verschwunden.

Die erfindungsgemäße Einrichtung zur Magnetotherapie benutzte man zum Kupieren von Phantomschmerzen bei einem Kranken mit linkem Unterschenkelstumpf. Man stellte eine Amplitude der Magnetfeldimpulse von 1500 kA/m, eine Impulsdauer von 3.10⁻⁴ s, eine Anstiegsgeschwindigkeit der Impulsvorderflanke von 7,5.10⁷ kA/ms ein. Nach der Einwirkung mit 55 Impulsen ( ∼ 2,7 min) trat beim Kranken ein Zustand subjektiver Betäubungsempfindung ein. Nach der Durchführung des ersten magnetotherepeutischen Behandlungsschrittes mit 70 Impulsen wurde der Schmerzbetäbungseffekt zwei Stunden lang beibehalten, nach der Durchführung des zweiten Behandlungsschrittes mit 70 Impulsen blieb der Schmerzbetäubungseffekt 10,5 Stunden erhalten und nach dem dritten Behandlungsschritt mit 70 Impulsen entstanden keine Phantomschmerzen mehr.

### Gewerbliche Anwendbarkeit

Die Erfindung kann erfolgreich auf verschiedenen Gebieten der Volkswirtschaft verwendet werden:
- in der Medizin zum Kupieren eines akuten schmerzhaften Syndroms sowie zur Erhöhung der Reaktivität des Organismus und Beschleunigung regenerativer Prozesse;
- in der Veterinärkunde zur Behandlung und Prophylaxe von Erkrankungen bei Tieren, besonders Mastitiden bei Rindern;
- in der Chemie als Katalysator in chemischen Vorgängen;
- in der Biologie bei Untersuchung der Möglichkeiten biologischer Objekte.

## Patentansprüche

1. Einrichtung zur Magnetotherapie, die - in Reihe geschaltet - einen steuerbaren Stromimpulsgenerator (1), einen Energiespeicher (2) und einen Entladungskreis für den Energiespeicher (3) enthält, welcher Entladungskreis ein steuerbares Schaltelement (5) und einen Induktor (4), durch den ein heilendes Impulsmagnetfeld erzeugbar ist, aufweist, und die mit einer Einheit (7) zur Steuerung der Magnetfeldimpulsdauer des Induktors (4), deren Ausgang an den Steuereingang des steuerbaren Schaltelementes (5) angeschlossen ist, und einem Magnetfeldimpulsamplitudenregler (10) versehen ist, dessen Ausgang an den Steuereingang des steuerbaren Stromimpulsgenerators (1) angeschlossen ist, **dadurch gekennzeichnet,** daß in den Entladungskreis (3) des Energiespeichers (2) ein Regelteil (6) für die Vorderflankensteilheit der Magnetfeldimpulse des Induktors (4) geschaltet ist.

2. Einrichtung zur Magnetotherapie nach Anspruch 1, **dadurch gekennzeichnet,** daß der Regelteil (6) für die Vorderflankensteilheit der Magnetfeldimpulse zwischen dem Ausgang des Energiespeichers (2) und dem Eingang des Schaltelementes (5) geschaltet ist und mindestens zwei LC-Ketten (23 bis 26), deren eine Anschlußenden vereinigt sind, und einen Wahlschalter ( 27) enthält, dessen Ruhekontakte mit den anderen Anschlußenden der entsprechenden LC-Ketten verbunden sind.

3. Einrichtung zur Magnetotherapie nach Anspruch 1, bei der der Induktor eine mehrwindige Induktivitätsspule darstellt, **dadurch gekennzeichnet,** daß der Regelteil für die Vorderflankensteilheit der Magnetfeldimpulse mit einem Induktor (22) konstruktiv vereinigt ist, wozu die Wicklung seiner Induktivitätsspule (30) mit einer Vielzahl von Abzweigen (31) über die Länge des Wicklungsleiters versehen und an den Ausgang des Schaltelementes (5) über einen Wahlschalter (33) angeschlossen ist, dessen jeder Ruhekontakt (38) mit dem zugehörigen Abzweig (31) verbunden ist.

4. Einrichtung zur Magnetotherapie nach einem der Ansprüche 1, 2, 3, bei welcher der Magnetfeldimpulsamplitudenregler (10) eine Vergleichsschaltung (46) enthält, deren erster Eingang an den Ausgang eines Magnetfeldimpulsamplitudengebers (12), zweiter Eingang an den Ausgang eines Magnetfeldimpulsamplitudeneinstellers (48) und deren Ausgang an den Ausgang des Magnetfeldimpulsamplitudenreglers (10) angeschlossen sind, **dadurch gekennzeichnet,** daß der Amplitudenregler (10) zusätzlich mit einer zwischen dem Ausgang des Magnetfeldimpulsamplitudengebers (12) und dem ersten Eingang der Vergleichsschaltung (26) geschalteten Speichereinheit (45) und einer zweiten Vergleichsschaltung (50) versehen ist, deren erster Eingang mit dem Ausgang der ersten Vergleichsschaltung (46), zweiter Eingang über einen Spannungsteiler (51) mit dem Ausgang des Energiespeichers (2) und deren Ausgang mit dem Ausgang des Amplitudenreglers (10) verbunden sind.

## Claims

1. Device for magnetotherapy, which contains, connected in series, a controllable current pulse generator (1), an energy store (2) and a discharge circuit for the energy store (2), which discharge circuit comprises a controllable switching element (5) and an inductor (4), by means of which a healing pulsed magnetic field can be generated, and which is provided with a unit (7) for controlling the duration of the magnetic field pulse of the inductor (4), the output of which is connected to the control input of the controllable switching element (5), and with a magnetic field pulse amplitude controller (10), the output of which is connected to the control input of the controllable current pulse generator (1), characterised in that a control part (6) for the front edge slope of the magnetic field pulses of the inductor (4) is connected in the discharge circuit (3) of the energy store (2).

2. Device for magnetotherapy according to Claim 1, characterised in that the control part (6) for the front edge slope of the magnetic field pulses is connected between the output of the energy store (2) and the input of the switching element (5) and contains at least two LC chains (23 to 26), the end connections on one side of which are joined, and a selector switch (27), the home contacts of which are connected to the other connecting ends of the corresponding LC chains.

3. Device for magnetotherapy according to Claim 1, in which the inductor is an inductance coil with multiple windings, characterised in that the control part for the front edge slope of the magnetic field pulses is structurally combined with an inductor (22), for which purpose the winding of its inductance coil (30) is provided with a multiplicity of taps (31) over the length of the winding conductor and is connected to the output of the switching element (5) through a selector switch (33), each home contact (8) of which is connected to the associated tap (31).

4. Device for magnetotherapy according to one of the Claims 1, 2, 3,in which the magnetic field pulse amplitude controller (10) contains a comparator circuit (46), the first input of which is connected to the output of a magnetic field pulse amplitude pickup (12),second input to the output of a magnetic field pulse amplitude adjuster (48) and the output of which is connected to the output of the magnetic field pulse amplitude controller (10), characterised in that the amplitude controller (10) is additionally provided with a memory unit(45) connected between the output of the magnetic field pulse amplitude pickup (12) and the first input of the comparator circuit (26), and a second comparator circuit (50), the first input of which is connected to the output of the first comparator circuit (46), the second input via a voltage divider (51) to the output of the energy store (2) and its output to the output of the amplitude controller (10).

## Revendications

1. Dispositif pour la magnétothérapie, qui contient, branchés en série, un générateur d'impulsions de courant commandé (1), un accumulateur d'énergie (2) et un circuit de décharge (3) pour l'accumulateur d'énergie, ce circuit de décharge présentant un élément de commutation (5) commandé et un inducteur (4) par lequel il est possible de créer un champ magnétique pulsé curatif, et qui est doté d'une unité (7) pour la commande de la durée des impulsions de champ magnétique de l'inducteur (4), dont la sortie est raccordée à l'entrée de commande de l'élément de commutation (5) commandé, ainsi qu'un régulateur (10) de l'amplitude des impulsions de champ magnétique, dont la sortie est raccordée à l'entrée de commande du générateur d'impulsions de courant commandé (1), caractérisé en ce qu'une partie de régulation (6) de la pente du flanc de montée des impulsions de champ magnétique de l'inducteur (4) est branchée dans le circuit de décharge (3) de l'accumulateur d'énergie (2).

2. Dispositif pour la magnétothérapie selon la revendication 1, caractérisé en ce que la partie de régulation (6) de la pente du flanc de montée des impulsions de champ magnétique est branchée entre la sortie de l'accumulateur d'énergie (2) et l'entrée de l'élément de commutation (5), et contient au moins deux circuits LC (23 à 26), présentant une borne de raccordement commune, ainsi qu'un commutateur de sélection (27) dont les contacts rupteurs sont reliés aux autres extrémités de raccordement du circuit LC correspondant.

3. Dispositif pour la magnétothérapie selon la revendication 1, dans lequel l'inducteur présente une bobine d'induction à plusieurs spires, caractérisé en ce que la partie de régulation de la pente du flanc de montée des impulsions de champ magnétique est intégrée par construction à un inducteur (22), en vue de quoi le bobinage de sa bobine d'induction (30) est doté de plusieurs dérivations (31) disposées sur la longueur du conducteur bobiné, et est raccordé à la sortie de l'élément de commutation (5) par l'intermédiaire d'un commutateur de sélection (33) dont chaque contact rupteur (38) est relié à la dérivation (31) associée.

4. Dispositif pour la magnétothérapie selon l'une des revendications 1, 2, 3, dans lequel le régulateur (10) de l'amplitude des impulsions de champ magnétique contient un circuit de comparaison (46) dont la première entrée est raccordée à la sortie d'un détecteur (12) d'amplitude des impulsions de champ magnétique, dont la seconde entrée est raccordée à la sortie d'un dispositif de réglage (48) de l'amplitude des impulsions de champ magnétique, et dont la sortie est raccordée à la sortie du régulateur (10) d'amplitude des impulsions de champ magnétique, caractérisé en ce que le régulateur d'amplitude (10) est de plus doté d'une unité d'accumulation (45) branchée entre la sortie du détecteur (12) d'amplitude des impulsions de champ magnétique et la première entrée du circuit de comparaison (26), ainsi que d'un second circuit de comparaison (50) dont la première entrée est reliée à la sortie du premier circuit de comparaison (46), dont la seconde entrée est reliée à la sortie de l'accumulateur d'énergie (2) par l'intermédiaire d'un diviseur de tension (51), et dont la sortie est reliée à la sortie du régulateur d'amplitude (10).
